# EUROPÄISCHE PATENTSCHRIFT

(11) **EP 3 253 980 B1**
(45) Veröffentlichungstag und Bekanntmachung des Hinweises auf die Patenterteilung: **08.04.2020**
(21) Anmeldenummer: 16714223.1
(22) Anmeldetag: 03.02.2016
(51) Int. Cl.: F16D 3/12, F16D 3/72, F16H 55/36

(54) **ENTKOPPLER**
DECOUPLER
DISPOSITIF DE DÉCOUPLAGE

(30) Priorität: 05.02.2015 DE 102015202043
(43) Veröffentlichungstag der Anmeldung: 13.12.2017
(73) Patentinhaber: Schaeffler Technologies AG & Co. KG, 91074 Herzogenaurach (DE)
(72) Erfinder: FARIA, Christof, 91054 Erlangen (DE)
(86) Internationale Anmeldenummer: PCT/DE2016/200068
(87) Internationale Veröffentlichungsnummer: WO 2016/124195

(56) Entgegenhaltungen:
- WO-A1-98/50709
- WO-A1-2006/081657
- WO-A1-2013/124009
- WO-A1-2016/037283
- CN-Y- 200 982 361
- US-A- 3 019 871
- US-A- 5 370 585
- US-B2- 8 047 920

## Beschreibung

Die Erfindung betrifft einen Entkoppler zur Übertragung eines Antriebsmoments von einem Drehantrieb auf einen Drehabtrieb, mit:
- einem im Antriebsmomentfluss seitens des Drehantriebs angeordneten Antriebsteil,
- einem im Antriebsmomentfluss seitens des Drehabtriebs angeordneten Abtriebsteil,
- einer im Antriebsmomentfluss zwischen dem Antriebsteil und dem Abtriebsteil angeordneten Reihenschaltung aus einer Schraubendrehfeder und einer Einwegkupplung, die in Antriebsdrehrichtung ein Überholen des Abtriebsteils gegenüber dem Antriebsteil zulässt,
- einem im Antriebsmomentfluss seitens des Antriebsteils angeordneten ersten Federteller für das erste Ende der Schraubendrehfeder
- und einem im Antriebsmomentfluss seitens des Abtriebsteils angeordneten zweiten Federteller für das zweite Ende der Schraubendrehfeder.
Die Federteller steigen axial rampenförmig an, und die daran anliegenden Schraubendrehfederenden weiten die Schraubendrehfeder unter Übertragung des Antriebsmoments radial auf.

Die Erfindung betrifft weiterhin einen Nebenaggregate-Riementrieb mit einem Entkoppler der genannten Art.

Derartige Entkoppler (Englisch: Decoupler) sind typischerweise als Riemenscheiben-Entkoppler eines Nebenaggregate-Riementriebs einer Brennkraftmaschine ausgebildet. Sie können als Kurbelwellen-Entkoppler auf der Kurbelwelle oder als Generator-Entkoppler auf dem Generator angeordnet sein und den Eintrag von Drehschwingungen und -ungleichförmigkeiten der Kurbelwelle in den Nebenaggregate-Riementrieb bzw. in den Generator kompensieren. Die Reihenschaltung aus der Einwegkupplung und der Schraubendrehfeder überträgt im geschlossenen Zustand der Einwegkupplung das Antriebsmoment vom Antriebsteil auf das Abtriebsteil, wobei die Elastizität der Schraubendrehfeder die Drehungleichförmigkeiten glättet. Bei verzögert rotierendem Antriebsteil öffnet die Einwegkupplung, wobei - dann umgekehrt - kein nennenswertes Drehmoment vom Abtriebsteil auf das Antriebsteil übertragen werden kann. Im Falle des Generator-Entkopplers kann dann die mit relativ großer Massenträgheit behaftete Welle des Generators dessen Riemenscheibe überholen.

Ein gattungsgemäßer Generator-Entkoppler geht beispielsweise aus der US 8,047,920 B2 hervor. Die Einwegkupplung ist als Schlingband ausgebildet, das in der Reihenschaltung antriebseitig und radial zwischen der Schraubendrehfeder und der Riemenscheibe angeordnet ist. Obwohl das Schlingband bei überholender Generatorwelle geöffnet ist, kann das Reibmoment zwischen dem Innenmantel der Riemenscheibe und dem daran anliegenden Schlingband zu einer Relativverdrehung der beiden Federteller führen, wobei sich die Enden der Schraubendrehfeder von den umfänglichen Federanlagestufen der rampenförmigen Federteller entfernen und an deren Rampen hochlaufen. Der dabei infolge der Rampengeometrie effektiv abnehmende Axialbauraum für die Schraubendrehfeder kann bewirken, dass die Schraubendrehfeder die beiden Federteller axial auseinander drückt und so den Riemenscheibenentkoppler gleichsam axial sprengt. Eine ebenso unerwünschte Konsequenz ist die auffällige Akustik des Entkopplers, wenn ein oder beide Federenden wiederholt an den Rampen hochlaufen und nach jeder Umdrehung an den Stufen zurück schnappen.

Als Lösung für dieses Problem schlägt die US 8,047,920 B2 einen Mechanismus vor, der die unerwünschte Relativverdrehung der beiden Federteller blockiert. Im Überholbetrieb laufen dann beide Federteller synchron und als Einheit mit der Schraubendrehfeder um und verhindern so den Rampenhochlauf der Federenden. Das Blockieren erfolgt konstruktiv durch Drehanschläge, die einerseits am Abtriebsteil und andererseits am antriebseitigen Federteller befestigt sind und im Überholbetrieb diesen Federteller mitnehmen.

Ausgehend hiervon liegt der vorliegenden Erfindung die Aufgabe zugrunde, einen Entkoppler der eingangs genannten Art mit einer alternativen Konstruktion anzugeben, die das Rampenhochlaufen der Schraubendrehfeder ebenfalls verhindert.

Die Lösung hierfür ergibt sich aus den Merkmalen des Anspruchs 1. Demnach sollen die Schraubendrehfederenden und die Federteller gegenseitige Drehanschläge haben, die jeweils in Antriebsdrehrichtung eine Relativverdrehung des zweiten Federtellers gegenüber dem zweiten Schraubendrehfederende und des ersten Schraubendrehfederendes gegenüber dem ersten Federteller verhindern.

Die Erfindung basiert auf dem Prinzip, dass die Schraubendrehfeder selbst die beiden Federteller miteinander drehkoppelt, um das unerwünschte Rampenhochlaufen der Federenden zu verhindern. Dies erfolgt konstruktiv dadurch, dass die Schraubendrehfeder nicht nur in der Richtung belastbar ist, in der sie unter radialer Aufweitung des Wicklungskörpers das Antriebsmoment überträgt. Vielmehr ist die Schraubendrehfeder auch in ausreichend hohem Maße in der dazu umgekehrten Momentenrichtung belastbar, in der sie sich radial zusammenzieht. Nur die Kombination aus der ausreichend großen Federbelastbarkeit in beide Drehmomentrichtungen erzwingt es im Überholbetrieb des Entkopplers, dass die Schraubendrehfeder und die beiden Federteller als Einheit umlaufen, so dass das den unerwünschten Rampenhochlauf bewirkende Überholen des zweiten Federtellers gegenüber dem ersten Federteller verhindert ist.

In Abhängigkeit der Positionierung der Schraubendrehfeder innerhalb der Reihenschaltung mit der Einwegkupplung können im Überholbetrieb des Entkopplers folgende Zustände auftreten:
- wenn die Schraubendrehfeder abtriebseitig, d.h. im Antriebsmomentfluss hinter der Einwegkupplung positioniert ist, dann nimmt der mit dem überholenden Abtrieb umlaufende, zweite Federteller das zweite Schraubendrehfederende mit und das erste Schraubendrehfederende nimmt den ersten Federteller entgegen der Reibung der geöffneten Einwegkupplung mit. Somit laufen im Überholbetrieb des Entkopplers beide Federteller und die Schraubendrehfeder ohne den unerwünschten Rampenhochlauf als Einheit um.
- wenn die Schraubendrehfeder antriebseitig, d.h. im Antriebsmomentfluss vor der Einwegkupplung positioniert ist, dann schlagen der abtriebseitige zweite Federteller am zweiten Schraubendrehfederende und das erste Schraubendrehfederende am ersten Federteller an. Die aus den Federtellern und der Schraubendrehfeder gebildete Einheit läuft mit dem überholten Drehantrieb entgegen der Reibung der geöffneten Einwegkupplung als Einheit ohne den unerwünschten Rampenhochlauf um.

Die Belastbarkeit der Schraubendrehfeder in beide Momentenrichtungen erfolgt vorzugsweise durch Drehanschläge, die jeweils voneinander lösbar und folglich einfach aneinander montierbar sind. Als Alternative zu formschlüssigen und lösbaren Verbindungen können die Drehanschläge aber auch jeweils unlösbar aneinander befestigt sein. In diesem Fall sind dann beispielsweise jeweils ein Federende und ein Federteller mittels einer Press- oder Schweißverbindung aneinander befestigt, die eine den Wicklungskörper radial zusammen ziehende Drehmomentbelastung der Schraubendrehfeder ermöglicht. Die beiden Drehanschläge seitens der Schraubendrehfeder sind vorzugsweise zueinander symmetrisch, so dass bei der Montage des Entkopplers eine gerichtete Einbaulage der Schraubendrehfeder nicht erforderlich ist.

Die Richtungsumkehr zwischen der den Federwicklungskörper radial aufweitenden und radial zusammen ziehenden Drehmomentbelastung kann sowohl mit geringem Übergangsspiel als auch spielfrei in Bezug auf die dann in bzw. außer gegenseitigen Eingriff kommenden Drehanschläge erfolgen.

Weitere Merkmale der Erfindung ergeben sich aus der nachfolgenden Beschreibung und den Zeichnungen, in denen die Erfindung prinzipiell und anhand von Ausführungsbeispielen dargestellt ist. Sofern nicht anders erwähnt, sind dabei gleiche oder funktionsgleiche Merkmale oder Bauteile mit gleichen Bezugszeichen versehen. Es zeigen:
- Figur 1: eine Prinzipdarstellung eines erfindungsgemäßen Generator-Entkopplers eines Nebenaggregate-Riementriebs einer Brennkraftma-schine;
- Figur 2: den Drehmomentfluss des Generatorentkopplers gemäß Figur 1 im Überholbetrieb;
- Figur 3: eine Prinzipdarstellung eines erfindungsgemäßen Kurbelwellen-Entkopplers eines Nebenaggregate-Riementriebs einer Brennkraftma-schine;
- Figur 4: die Schraubendrehfeder eines ersten Ausführungsbeispiels in perspek-tivischer Ansicht;
- Figur 5: einen zur Schraubendrehfeder des ersten Ausführungsbeispiels zuge-hörigen Federteller in perspektivischer Ansicht;
- Figur 6: die Schraubendrehfeder eines zweiten Ausführungsbeispiels in per-spektivischer Ansicht;
- Figur 7: einen zur Schraubendrehfeder des zweiten Ausführungsbeispiels zuge-hörigen Federteller in perspektivischer Ansicht;
- Figur 8: einen zur Schraubendrehfeder des zweiten Ausführungsbeispiels zuge-hörigen, alternativen Federteller in perspektivischer Ansicht;
- Figur 9: die Schraubendrehfeder eines dritten Ausführungsbeispiels in perspek-tivischer Ansicht;
- Figur 10: einen zur Schraubendrehfeder des dritten Ausführungsbeispiels zuge-hörigen Federteller in perspektivischer Ansicht;
- Figur 11: die Schraubendrehfeder eines vierten Ausführungsbeispiels gemein-sam mit einer Einwegkupplung und einem zugehörigen Abtriebsteil mit integralem Federteller in perspektivisch explodierter Darstellung.

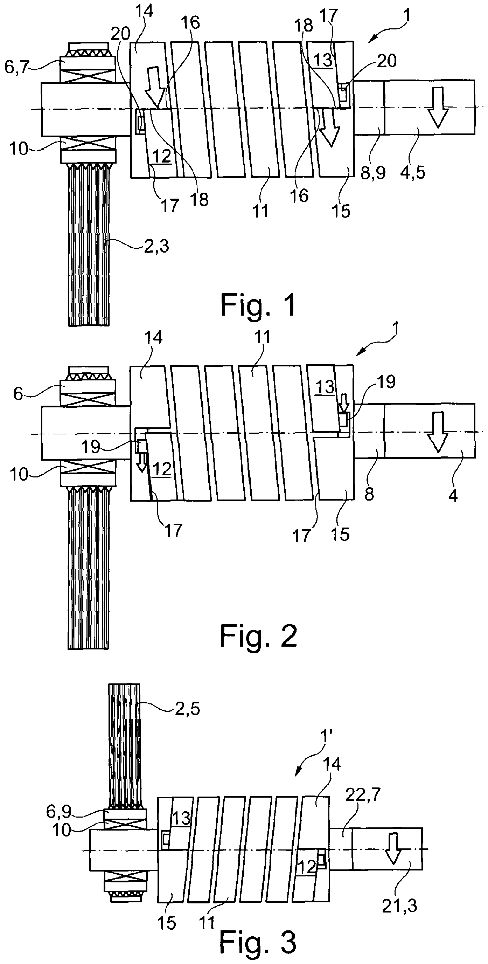

Figur 1 zeigt eine Prinzipdarstellung eines auf dem Generator eines Nebenaggregate-Riementriebs einer Brennkraftmaschine angeordneten Entkopplers 1. Dieser überträgt das Antriebsmoment des Riemens 2 als Drehantrieb 3 auf die Generatorwelle 4 als Drehabtrieb 5 und umfasst folgende Komponenten im Antriebsmomentfluss:
- eine vom Riemen 2 umschlungene Riemenscheibe 6 als antriebseitig angeordnetes Antriebsteil 7,
- eine auf der Generatorwelle 4 befestigte Nabe 8 als abtriebseitig angeordnetes Abtriebsteil 9,
- eine zwischen der Riemenscheibe 6 und der Nabe 8 angeordnete Reihenschaltung aus einer Einwegkupplung 10 und einer Schraubendrehfeder 11, deren erstes Ende 12 seitens der Riemenscheibe 6 und deren zweites Ende 13 seitens der Nabe 8 verläuft,
- einen ersten Federteller 14 für das erste Schraubendrehfederende 12 und
- einen zweiten Federteller 15 für das zweite Schraubendrehfederende 13.

Der Antrieb des Generators erfolgt in der auf der Generatorwelle 4 eingezeichneten Drehrichtung, d.h. im Uhrzeigersinn, wenn man in der Figur von links auf den Riementrieb sieht.

Die der elastischen Übertragung des Antriebsmoments von der Riemenscheibe 6 auf die Generatorwelle 4 dienende Schraubendrehfeder 11 ist zwischen dem antriebseitigen ersten Federteller 14 und dem abtriebseitigen zweiten Federteller 15 sowohl umfänglich als auch mit leichter axialer Vorspannung eingespannt. Der erste Federteller 14 ist sowohl gegenüber der Riemenscheibe 6 als auch gegenüber der Nabe 8 drehbar und wird lediglich durch die geschlossene Einwegkupplung 10 drehfest mit der Riemenscheibe 6 verbunden. Der zweite Federteller 15 ist drehfest mit der Nabe 8 verbunden. Beide Federteller 14, 15 steigen jeweils (über dem Umfang ihrer Stirnseiten) axial rampenförmig an und sind somit im wesentlichen komplementär zu den Schraubendrehfederenden 12, 13 geformt, die an den Federtellern 14 bzw. 15 anliegen. Die Übertragung des Antriebsmoments erfolgt an beiden Schraubendrehfederenden 12, 13 jeweils durch Druckkontakt zwischen den Stirnseiten 16 des Schraubendrehfederendes 12, 13 und einer durch die axiale Rampe 17 jedes Federtellers 14, 15 gebildeten Stufe 18, so dass der Wicklungskörper der Schraubendrehfeder 11 unter der Antriebsmomentbelastung von deren Enden 12, 13 radial aufgeweitet wird.

Die in Figur 1 an den Federtellern 14, 15 eingezeichneten Pfeile symbolisieren den Antriebsmomentfluss im Entkoppler 1, wenn die Riemenscheibe 6 bei geschlossener Einwegkupplung 10 die Nabe 8 in Antriebsdrehrichtung antreibt. Dabei wird das Antriebsmoment einerseits von der Stufe 18 des ersten Federtellers 14 auf die Stirnseite 16 des ersten Schraubendrehfederendes 12 und andererseits von der Stirnseite 16 des zweiten Schraubendrehfederendes 13 auf die Stufe 18 des zweiten Federtellers 15 übertragen.

Figur 2 zeigt den anderen Betriebszustand des Entkopplers 1, in dem die (träge) Generatorwelle 4 in der darauf eingezeichneten Drehrichtung die Riemenscheibe 6 überholt. Ein unzulässiges Hochlaufen eines oder beider Schraubendrehfederenden 12, 13 an den Rampen 17 der Federteller 14, 15 wird durch gegenseitige Drehanschläge 19, 20 an beiden Schraubendrehfederenden 12, 13 bzw. an beiden Federtellern 14, 15 verhindert. Die Drehanschläge 19 seitens der Schraubendrehfeder 11 sind jeweils als axiale Vorsprünge an deren Enden 12, 13 gebildet, und die Drehanschläge 20 seitens der Federteller 14, 15 sind jeweils durch axiale Ausnehmungen (s. Figur 1) gebildet, in die die Vorsprünge hinein ragen.

In Analogie zu Figur 1 symbolisieren die in Figur 2 eingezeichneten Pfeile den Drehmomentfluss im Entkoppler 1, wenn die Riemenscheibe 6 bei geöffneter Einwegkupplung 10 von der Nabe 8 in Antriebsdrehrichtung überholt wird. Dabei wird ein die Schraubendrehfeder 11 mitnehmendes Drehmoment einerseits vom Drehanschlag 20 des zweiten Federtellers 15 auf den Drehanschlag 19 des zweiten Schraubendrehfederendes 13 und andererseits vom Drehanschlag 19 des ersten Schraubendrehfederendes 12 auf den Drehanschlag 20 des ersten Federtellers 14 übertragen. Der Fluss dieses Mitnahmemoments, dessen Größe von der Kontaktreibung der geöffneten Einwegkupplung 10 mit deren Kontaktpartnern abhängt, erzwingt einen gemeinsamen Umlauf der beiden Federteller 14, 15 mit der das Mitnahmemoment selbst übertragenden Schraubendrehfeder 11. Da auch dann die Federteller 14, 15 lediglich im Rahmen der Drehelastizität der Schraubendrehfeder 11 relativ zueinander verdrehbar sind, wird der unerwünschte Hochlauf der Schraubendrehfederenden 12, 13 an den Rampen 17 stets verhindert.

Figur 3 zeigt eine Prinzipdarstellung eines Entkopplers 1', der den Nebenaggregate-Riementrieb einer Brennkraftmaschine antreibt. In diesem Fall ist die Kurbelwelle 21 der Drehantrieb 3, und der Riemen 2 ist der Drehabtrieb 5. Der Entkoppler 1' umfasst folgende Komponenten im Antriebsmomentfluss:
- eine an der Kurbelwelle 21 befestigte Welle 22 als antriebseitig angeordnetes Antriebsteil 7,
- eine vom Riemen 2 umschlungene Riemenscheibe 6 als abtriebseitig angeordnetes Abtriebsteil 9,
- eine zwischen der Welle 22 und der Riemenscheibe 6 angeordnete Reihenschaltung aus einer Schraubendrehfeder 11 und einer Einwegkupplung 10, wobei das erste Schraubendrehfederende 12 seitens der Welle 22 und das zweite Schraubendrehfederende 13 seitens der Riemenscheibe 6 verläuft,
- einen ersten Federteller 14 für das erste Schraubendrehfederende 12 und
- einen zweiten Federteller 15 für das zweite Schraubendrehfederende 13.

Der Antrieb des Riemens 2 erfolgt in der auf der Kurbelwelle 21 eingezeichneten Drehrichtung, d.h. ebenfalls im Uhrzeigersinn, wenn man in der Figur von links auf den Riementrieb sieht. Da gegenüber Figur 1 der Drehantrieb und -abtrieb von links nach rechts vertauscht angeordnet sind, hat auch die Schraubendrehfeder 11 eine demgegenüber umgekehrte Wickelrichtung. In Bezug auf die erfindungsgemäße Verhinderung des Rampenhochlaufs mittels der Drehanschläge 19, 20 gelten jedoch die vorstehenden Erläuterungen der Figuren 1 und 2 analog.

In den Figuren 1 bis 3 kann die Reihenfolge der die Reihenschaltung bildenden Einwegkupplung 10 und Schraubendrehfeder 11 bezüglich des Antriebsmomentflusses auch vertauscht sein. Im Überholbetrieb würden dann die jeweils aus den Federtellern 14, 15 und der Schraubendrehfeder 11 gebildeten Einheiten synchron mit der Riemenscheibe 6 umlaufen. Außerdem kann das an den Drehanschlägen 19, 20 eingezeichnete Umfangsspiel der Schraubendrehfederenden 12, 13 auch eliminiert sein.

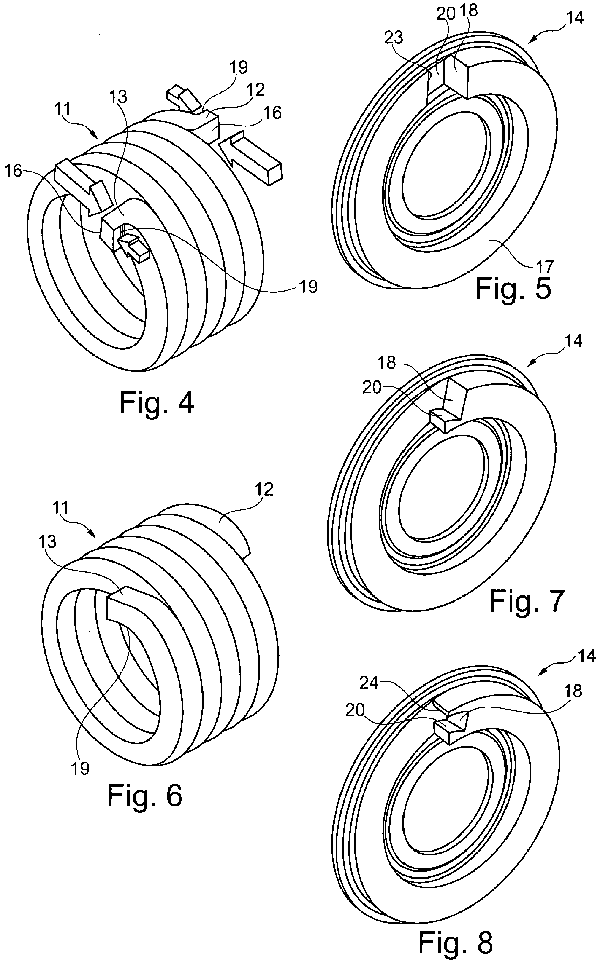

Die in Figur 4 dargestellte Schraubendrehfeder 11 und ein Federteller 14 gemäß Figur 5 stellen ein erstes Ausführungsbeispiel erfindungsgemäßer Drehanschläge 19, 20 dar. Beide Enden 12, 13 der aus einem Federdraht mit Rechteckquerschnitt gewickelten Schraubendrehfeder 11 sind axial auswärts abgewinkelt. Der Federteller 14 hat im Bereich der durch die axiale Rampe 17 gebildeten Stufe 18 eine axiale Ausnehmung als Drehanschlag 20, in die der abgewinkelte Teil des Schraubendrehfederendes 12 als Drehanschlag 19 hineinragt und an der umfänglichen Begrenzung 23 der Ausnehmung 20 anschlägt.

Die in Figur 4 eingezeichneten Pfeilpaare symbolisieren den Drehmomenteintrag in die Schraubendrehfederenden 12, 13. Die deren Stirnseiten 16 beaufschlagenden Pfeile kennzeichnen das Antriebsmoment, das von der sich dabei radial aufweitenden Schraubendrehfeder 11 vom Drehantrieb auf den Drehabtrieb elastisch übertragen wird. Die kleinen Pfeile beaufschlagen die als federseitige Drehanschläge 19 dienenden Rückseiten der Stirnseiten 16 und kennzeichnen das Mitnahmemoment, das von der sich dabei radial zusammen ziehenden Schraubendrehfeder 11 vom abtriebseitigen zweiten Federteller 15 auf den antriebseitigen ersten Federteller 14 übertragen wird.

Die in Figur 6 dargestellte Schraubendrehfeder 11 bildet mit dem Federteller 14 gemäß Figur 7 ein zweites Ausführungsbeispiel der Erfindung. In diesem Fall sind die federseitigen Drehanschläge 19 durch die radialen Innenseiten der Schraubendrehfederenden 12, 13 gebildet, die radial einwärts abgewinkelt sind und in Bezug auf den zylindrischen Wicklungskörper der Schraubendrehfeder 11 etwa sekantenartig auslaufen. Der zugehörige federtellerseitige Drehanschlag 20 ist durch einen inneren Absatz gebildet, der - jeweils in Federumfangsrichtung - von der Stufe 18 ausgeht und im wesentlichen komplementär zum Schraubendrehfederende 12 geneigt ist und dieses radial innen hintergreift. Dieser Hintergriff ermöglicht den Eintrag des die Schraubendrehfeder 11 radial zusammen ziehenden Mitnahmemoments.

Der in Figur 8 dargestellte Federteller 14 ist eine Variante des Federtellers 14 gemäß Figur 7. Bei dieser Ausführung gehen von der Stufe 18 sowohl der innere Absatz 20 als auch ein äußerer Absatz 24 in Federumfangsrichtung aus. Der äußere Absatz 24 verläuft im wesentlichen mit der Federdrahtstärke äquidistant zum inneren Absatz 20 und verhindert ein radial auswärtiges Ausweichen des zwischen den beiden Absätzen 20, 24 eingespannten Schraubendrehfederendes 12, wenn an diesem die das Mitnahmemoment erzeugende Zugkraft angreift.

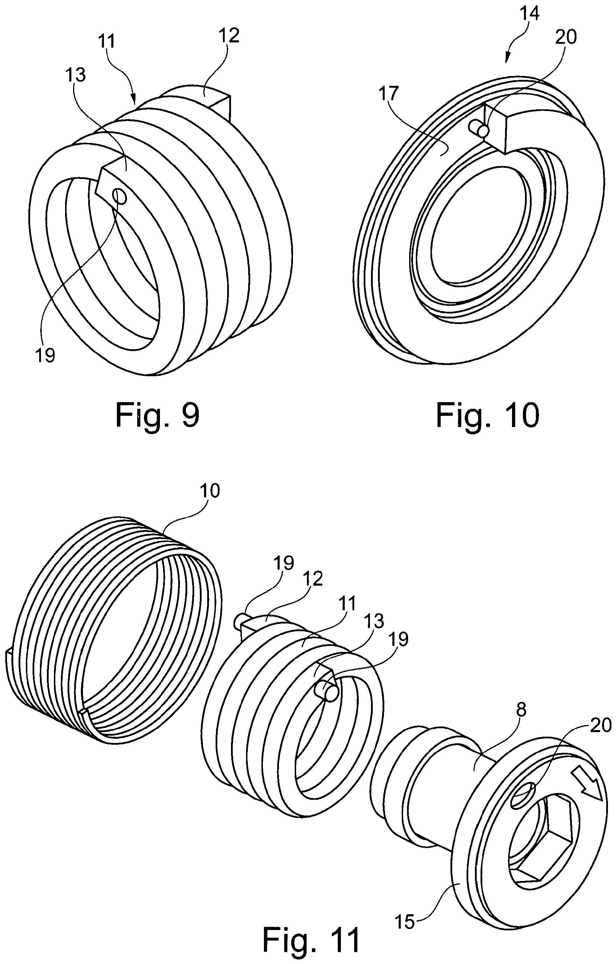

Beim dritten Ausführungsbeispiel gemäß den Figuren 9 und 10 sind die erfindungsgemäßen Drehanschläge 19, 20 seitens der Schraubendrehfeder 11 durch axiale Ausnehmungen in den Schraubendrehfederenden 12, 13 und seitens der Federteller 14, 15 durch axiale Vorsprünge gebildet, die sich von den Rampen 17 erheben und in die Ausnehmungen 19 hinein ragen.

Figur 11 zeigt als viertes Ausführungsbeispiel eine dazu vertauschte Anordnung der Drehanschläge 19, 20, die den Ausführungen gemäß den Figuren 1 bis 3 entsprechen. Die Drehanschläge 19, 20 sind seitens der Schraubendrehfeder 11 durch die axialen Vorsprünge an den Schraubendrehfederenden 12, 13 und seitens der Federteller 14, 15 durch axiale Ausnehmungen in den Rampen 17 gebildet, in die die Vorsprünge 19 hinein ragen. Die explodierte Darstellung zeigt linksseitig der Schraubendrehfeder 11 die Einwegkupplung 10 und rechtsseitig der Schraubendrehfeder 11 die Nabe 8 des Generator-Entkopplers 1. Die Einwegkupplung 10 ist ein antriebseitig positioniertes Schlingband und die auf der Generatorwelle 4 zu verschraubende Nabe 8 ist integraler Teil des (abtriebseitigen) zweiten Federtellers 15.

Als Alternative zu den dargestellten Drehanschlägen 19, 20 der Schraubendrehfederenden 12, 13 und der Federteller 14, 15 kommen eine Vielzahl weiterer Ausgestaltungen in Betracht, solange die Drehanschläge die Schraubendrehfederenden mit dem die Schraubendrehfeder radial zusammen ziehenden Mitnahmemoment beaufschlagen können. Solche Alternativen sind beispielsweise:
- nicht-kreisförmige Vorsprünge und/oder Ausnehmungen
- radial orientierte Vorsprünge und Ausnehmungen
- radial auswärts abgewinkelte Schraubendrehfederenden
- radiale oder axiale Abwinklungen der Schraubendrehfederenden mit Winkel >90° und <180°

### Bezugszeichen liste

- 1: Entkoppler
- 2: Riemen
- 3: Drehantrieb
- 4: Generatorwelle
- 5: Drehabtrieb
- 6: Riemenscheibe
- 7: Antriebsteil
- 8: Nabe
- 9: Abtriebsteil
- 10: Einwegkupplung
- 11: Schraubendrehfeder
- 12: erstes Ende der Schraubendrehfeder
- 13: zweites Ende der Schraubendrehfeder
- 14: erster Federteller
- 15: zweiter Federteller
- 16: Stirnseite eines Schraubendrehfederendes
- 17: Rampe eines Federtellers
- 18: Stufe
- 19: Drehanschlag eines Schraubendrehfederendes
- 20: Drehanschlag eines Federtellers
- 21: Kurbelwelle
- 22: Welle
- 23: Begrenzung der axialen Ausnehmung eines Federtellers
- 24: äußerer Absatz eines Federtellers

## Patentansprüche

1. Entkoppler zur Übertragung eines Antriebsmoments von einem Drehantrieb (3) auf einen Drehabtrieb (5), mit:
- einem im Antriebsmomentfluss seitens des Drehantriebs (3) angeordneten Antriebsteil (7),
- einem im Antriebsmomentfluss seitens des Drehabtriebs (5) angeordneten Abtriebsteil (9),
- einer im Antriebsmomentfluss zwischen dem Antriebsteil (7) und dem Abtriebsteil (9) angeordneten Reihenschaltung aus einer Schraubendrehfeder (11) und einer Einwegkupplung (10), die in Antriebsdrehrichtung ein Überholen des Abtriebsteils (9) gegenüber dem Antriebsteil (7) zulässt,
- einem im Antriebsmomentfluss seitens des Antriebsteils (7) angeordneten ersten Federteller (14) für das erste Ende (12) der Schraubendrehfeder (11)
- und einem im Antriebsmomentfluss seitens des Abtriebsteils (9) angeordneten zweiten Federteller (15) für das zweite Ende (13) der Schraubendrehfeder (11),
wobei die Federteller (14, 15) axial rampenförmig ansteigen und wobei die daran anliegenden Schraubendrehfederenden (12, 13) die Schraubendrehfeder (11) unter Übertragung des Antriebsmoments radial aufweiten, **dadurch gekennzeichnet, dass** die Schraubendrehfederenden (12, 13) und die Federteller (14, 15) gegenseitige Drehanschläge (19, 20) haben, die jeweils in Antriebsdrehrichtung eine Relativverdrehung des zweiten Federtellers (15) gegenüber dem zweiten Schraubendrehfederende (13) und des ersten Schraubendrehfederendes (12) gegenüber dem ersten Federteller (14) dadurch verhindern, dass bei geöffneter Einwegkupplung (10) ein die Schraubendrehfeder (11) mitnehmendes Drehmoment abtriebseitig vom Drehanschlag (20) des zweiten Federtellers (15) auf den Drehanschlag (19) des zweiten Schraubendrehfederendes (13) und antriebseitig vom Drehanschlag (19) des ersten Schraubendrehfederendes (12) auf den Drehanschlag (20) des ersten Federtellers (14) übertragen wird.

2. Entkoppler nach Anspruch 1, **dadurch gekennzeichnet, dass** die Drehanschläge (19, 20) seitens der Schraubendrehfeder (11) durch axial auswärts abgewinkelte Schraubendrehfederenden (12, 13) und seitens der Federteller (14, 15) durch die umfänglichen Begrenzungen (23) axialer Ausnehmungen gebildet sind, in die die Schraubendrehfederenden (12, 13) hinein ragen.

3. Entkoppler nach Anspruch 1, **dadurch gekennzeichnet, dass** die Drehanschläge (19, 20) seitens der Schraubendrehfeder (11) durch radial einwärts abgewinkelte Schraubendrehfederenden (12, 13) und seitens der Federteller (14, 15) durch innere Absätze gebildet sind, die die Schraubendrehfederenden (12, 13) radial innen hintergreifen.

4. Entkoppler nach Anspruch 3, **dadurch gekennzeichnet, dass** die Schraubendrehfederenden (12, 13) radial außen gegen äußere Absätze (24) der Federteller (14, 15) abgestützt sind.

5. Entkoppler nach Anspruch 4, **dadurch gekennzeichnet, dass** die äußeren Absätze (24) im wesentlichen äquidistant zu den inneren Absätzen verlaufen.

6. Entkoppler nach Anspruch 1, **dadurch gekennzeichnet, dass** die Drehanschläge (19, 20) seitens der Schraubendrehfeder (11) durch axiale Ausnehmungen in den Schraubendrehfederenden (12, 13) und seitens der Federteller (14, 15) durch axiale Vorsprünge gebildet sind, die in die Ausnehmungen hinein ragen.

7. Entkoppler nach Anspruch 1, **dadurch gekennzeichnet, dass** die Drehanschläge (19, 20) seitens der Schraubendrehfeder (11) durch axiale Vorsprünge an den Schraubendrehfederenden (12, 13) und seitens der Federteller (14, 15) durch axiale Ausnehmungen gebildet sind, in die die Vorsprünge hinein ragen.

8. Nebenaggregate-Riementrieb einer Brennkraftmaschine, umfassend einen Entkoppler (1) nach einem der vorhergehenden Ansprüche und einen vom Entkoppler (1) angetriebenen Generator mit einer Generatorwelle (4), **dadurch gekennzeichnet, dass**:
- der Drehantrieb (3) der Riemen (2) des Riementriebs ist,
- das Antriebsteil (7) eine Riemenscheibe (6) ist,
- der Drehabtrieb (5) die Generatorwelle (4) ist und
- das Abtriebsteil (9) eine auf der Generatorwelle (4) zu befestigende Nabe (8) ist.

## Claims

1. A decoupler for transferring a drive torque from a rotary drive (3) to a rotary output (5), comprising:
- a drive part (7) arranged in the drive torque flow on the rotary drive (3) side,
- an output part (9) arranged in the drive torque flow on the rotary output (5) side,
- a series connection which consists of a helical torsion spring (11) and a one-way clutch (10), arranged in the drive torque flow between the drive part (7) and the output part (9), which permits an overrunning of the output part (9) with respect to the drive part (7) in the drive direction of rotation,
- a first spring seat (14), arranged in the drive torque flow on the drive part (7) side, for the first end (12) of the helical torsion spring (11)
- and a second spring seat (15), arranged in the drive torque flow on the output part (9) side, for the second end (13) of the helical torsion spring (11),
wherein the spring seats (14, 15) increase axially in the manner of ramps and wherein the helical torsion spring ends (12, 13) bearing there against radially expand the helical torsion spring (11) while transferring the drive torque, **characterised in that** the helical torsion spring ends (12, 13) and the spring seats (14, 15) have reciprocal rotary stops (19, 20), which each, in the drive direction of rotation, prevent a relative torsion of the second spring seat (15) relative to the second helical torsion spring end (13) and of the first helical torsion spring end (12) relative to the first spring seat (14) such that, when the one-way clutch (10) is open, a torque entraining the helical torsion spring (11) is transferred on the output side from the rotary stop (20) of the second spring seat (15) to the rotary stop (19) of the second helical torsion spring end (13) and is transferred on the drive side from the rotary stop (19) of the first helical torsion spring end (12) to the rotary stop (20) of the first spring seat (14).

2. The decoupler according to claim 1, **characterised in that** the rotary stops (19, 20) are formed on the helical torsion spring (11) side by axially outwardly angled helical torsion spring ends (12, 13) and on the spring seats (14, 15) side by the circumferential boundaries (23) of axial recesses, into which the helical torsion spring ends (12, 13) protrude.

3. The decoupler according to claim 1, **characterised in that** the rotary stops (19, 20) are formed on the helical torsion spring (11) side by radially inwardly angled helical torsion spring ends (12, 13) and on the spring seats (14, 15) side by internal shoulders, which the helical torsion spring ends (12, 13) engage behind radially inside.

4. The decoupler according to claim 3, **characterised in that** the helical torsion spring ends (12, 13) are supported radially outwardly against outer shoulders (24) of the spring seats (14, 15).

5. The decoupler according to claim 4, **characterised in that** the outer shoulders (24) extend substantially equidistantly from the inner shoulders.

6. The decoupler according to claim 1, **characterised in that** the rotary stops (19, 20) are formed on the helical torsion spring (11) side by axial recesses in the helical torsion spring ends (12, 13) and on the spring seats (14, 15) side by axial projections which protrude into the recesses.

7. The decoupler according to claim 1, **characterised in that** the rotary stops (19, 20) are formed on the helical torsion spring (11) side by axial projections on the helical torsion spring ends (12, 13) and on the spring seats (14, 15) side by axial recesses, into which the projections protrude.

8. An auxiliary unit belt drive of an internal combustion engine, comprising a decoupler (1) according to one of the preceding claims and a generator which is driven by the decoupler (1) and has a generator shaft (4), **characterised in that**:
- the rotary drive (3) is the belt (2) of the belt drive,
- the drive part (7) is a pulley (6),
- the rotary output (5) is the generator shaft (4), and
- the output part (9) is a hub (8) to be fastened on the generator shaft (4).

## Revendications

1. Dispositif de découplage pour transmettre un couple d'entraînement d'un entraînement rotatif (3) à un entraînement rotatif (5), comprenant :
- une partie d'entraînement (7) agencée dans le flux de couple d'entraînement côté partie de l'entraînement rotatif (3),
- une partie d'entraînement (9) agencée dans le flux de couple d'entraînement côté partie de l'entraînement rotatif (5),
- un circuit en série constitué d'un ressort hélicoïdal (11) et d'un embrayage unidirectionnel (10) agencé dans le flux de couple d'entrainement entre la partie d'entraînement (7) et la partie d'entrainement (9) et permettant à la partie d'entrainement (9) de dépasser la partie d'entraînement (7) dans le sens de rotation d'entraînement ;
- une première plaque à ressort (14) agencée dans le flux de couple d'entraînement côté partie d'entraînement (7) pour la première extrémité (12) du ressort hélicoïdal (11)
- et une deuxième plaque à ressort (15) agencée dans le flux de couple d'entraînement côté partie d'entrainement (9) pour la deuxième extrémité (13) du ressort hélicoïdal (11),
dans lequel les plaques à ressort (14, 15) montent axialement en forme de rampe et dans lequel les extrémités du ressort hélicoïdal (12, 13) dilatent radialement le ressort hélicoïdal (11) lors de la transmission du couple d'entraînement, **caractérisé en ce que** les extrémités du ressort hélicoïdal (12, 13) et les plaques à ressort (14, 15) présentent des butées de rotation mutuelles (19, 20), qui empêchent, dans le sens de rotation d'entrainement, une rotation relative de la deuxième plaque de ressort (15) par rapport à la deuxième extrémité de ressort hélicoïdal (13) et la première extrémité de ressort hélicoïdal (12) par rapport à la première plaque de ressort (14), respectivement, de sorte que lors d'un embrayage unidirectionnel ouvert (10), un couple de rotation entraînant le ressort hélicoïdal (11) soit transféré côté mené de la butée de rotation (20) de la deuxième plaque de ressort (15) à la butée de rotation (19) du deuxième ressort hélicoïdal (13) et côté entraînement de la butée de rotation (19) du premier ressort hélicoïdal (12) à la butée de rotation (20) de la première plaque de ressort (14).

2. Dispositif de découplage selon la revendication 1, **caractérisé en ce que** les butées de rotation (19, 20) sont formées côté ressort hélicoïdal (11) par les ressort hélicoïdaux (12, 13) inclinés suivant un angle axialement vers l'extérieur et côté plaque de ressort (14, 15) par les contours (23) périphériques de saillies axiales, dans lesquelles les extrémités de ressort hélicoïdal (12, 13) font saillie.

3. Dispositif de découplage selon la revendication 1, **caractérisé en ce que** les butées de rotation (19, 20) sont formées côté ressort hélicoïdal (11) par les extrémités de ressort hélicoïdal (12, 13) inclinées suivant un angle radialement vers l'intérieur et côté plaque à ressort (14, 15) par des paliers intérieurs, qui s'engagent radialement à l'intérieur avec les extrémités de ressort hélicoïdal (12, 13).

4. Dispositif de découplage selon la revendication 3, **caractérisé en ce que** les extrémités de ressort hélicoïdal (12, 13) s'appuient radialement vers l'extérieur contre des paliers extérieurs (24) des plaques de ressort (14, 15).

5. Dispositif de découplage selon la revendication 4, **caractérisé en ce que** les paliers extérieurs (24) sont sensiblement équidistants des paliers intérieurs.

6. Dispositif de découplage selon la revendication 1, **caractérisé en ce que** les butées de rotation (19, 20) sont formées côté ressort hélicoïdal (11) par des évidements axiaux dans les extrémités de ressort hélicoïdal (12, 13) et côté plaque de ressort (14, 15) par des saillies axiales qui s'engagent dans les évidements.

7. Dispositif de découplage selon la revendication 1, **caractérisé en ce que** les butées de rotation (19, 20) sont formées côté ressort hélicoïdal (11) par des saillies axiales sur les extrémités de ressort hélicoïdal (12, 13) et côté plaque de ressort (14, 15) par des évidement axiaux qui s'engagent dans les saillies.

8. Transmission par courroie auxiliaire d'un moteur à combustion interne comprenant un dispositif de découplage (1) selon l'une des revendications précédentes et un générateur entraîné par le dispositif de découplage (1) avec un arbre de générateur (4), **caractérisé en ce que** :
- l'entraînement rotatif (3) de la courroie (2) est l'entraînement par courroie,
- la partie d'entraînement (7) est une poulie (6),
- l'entraînement rotatif (5) est l'arbre de générateur (4) et
- la partie d'entrainement (9) est un moyeu (8) destiné à être monté sur l'arbre de générateur (4).
